# EUROPEAN PATENT APPLICATION

(11) **EP 1 661 552 A1**
(43) Date of publication of application: **31.05.2006**
(21) Application number: 05107560.4
(22) Date of filing: 17.08.2005
(51) Int. Cl.: A61K 8/73, A61K 8/19, A61K 8/23, A61Q 1/00, A61Q 1/06, A61Q 5/00, A61Q 11/00, A61Q 15/00, A61Q 19/00

(54) **Solid cosmetic composition for topical use**

(30) Priority: 10.09.2004 IT MI20041731
(71) Applicant: SA.FO. S.p.A, 20083 Gaggiano (MI) (IT)
(72) Inventor: Bernardi, Paolo Ferdinando, 20090 Vimodrone (MI) (IT)
(74) Representative: Frignoli, Luigi

(57) **Abstract**

A solid cosmetic composition for topical use, for treating the skin, the scalp, the hair, the teeth and the mucous membranes, for decorating and making-up the skin, and for making-up, shaping or setting keratin fibres such as eyelashes, eyebrows and the hair, comprising gellan gum in combination with a second hydrocolloid.

## Description

The present invention relates to a solid cosmetic composition for treating the hair, teeth, mucous membranes, for decorating and making-up the skin, and for making-up, shaping or setting keratin fibres such as eyelashes, eyebrows and the hair.

Solid cosmetic products for treating the human body are widely available and are also used for cleaning, refreshing and shaping parts of the human body (for example the hair, eyelashes and eyebrows) or for changing or decorating the appearance of the skin and the hair, eyelashes and eyebrows.

A very large number of cosmetic compositions are known for this purpose, the most recent of which have the following compositions: gellan gum; hydrocolloids chosen from the group comprising xanthan gum, carboxymethyl cellulose, hydroxypropyl cellulose, hydroxypropylmethyl cellulose, hydroxymethyl cellulose, agar-agar, carrageen, alginates, carob gum, guar gum, gum arabic, karaya gum, tragacanth gum, ghatti gum, pectin, gelatin, caseinates, hydroxypropyl guar (to which reference will be made hereinafter for simplicity by using the term "hydrocolloids"); one or more alkaline or alkaline-earth metal salts with Cl⁻, SO₄⁻⁻, CO₃⁻⁻, PO₄⁻⁻ ions, borates and acetates.

Obviously, the cosmetic compositions always contain water and at least one cosmetically acceptable component chosen from the group comprising preservatives, antioxidants, UV filters, emollients, pigments, colorants, surfactants, emulsifiers, oils, greases, perfumes, deodorants, antiperspirants, solvent extracts, powders, decorative particles (for example of the type known as "Reflects glittering gold") and the like (hereinafter called simply "cosmetically acceptable component"). Other components can also be present, such as acids (including citric acid and succinic acid), saponified fatty acids, etc.

US 5928655 and the corresponding EP 0803245 describe solid cosmetic compositions comprising thermoreversible polysaccharides (including gellan gum), at least one wetting compound from 4 to 40%), and from 2% to 35% of a compound in powder form, plus water. As the thermoreversible polysaccharides and in particular the gellan gum are unsuitable alone for forming shaped cylinders or sticks contained in suitable containers (from which they have to be made to emerge and retract for daily use), for measured application to the skin, according to U 5928655 a powder filler is added to the composition to give it an adequate consistency and ability to deposit the cosmetic on the skin (make-up capability). However, the presence of the powder may leave visible traces and causes and unpleasant sensation when the composition is applied to the skin. Moreover the presence of the powder renders the composition opaque and non-transparent and hence unpleasant to the eye, whereas it is known that it is desirable to have a solid composition which does not leave too visible traces on the skin to which it is applied. If the powder is removed, a composition is obtained which is neither sufficiently solid nor sufficiently stable, and does not enable a thin layer or film of the composition to be satisfactorily deposited on the user's skin. All these drawbacks are well known and are highlighted in the introduction to the description of US 6280750 according to which, to obtain a cosmetic composition having the desired consistency characteristics, at least one hydrocolloid and at least one amphiphilic polymer (a compound having at least one fatty and hence hydrophobic chain) are added to the gellan gum (which can be present even in very large quantity, up to 15% by weight), these making the composition only slightly transparent and extrudable with difficulty. It should be noted that a quantity of more than 2% of gellan gum also makes the composition opaque.

The cosmetic composition described in US 6180122 has to comprise gellan gum in a quantity greater than 2% (and up to 15% by weight) and at least one other hydrocolloid (for a total which can reach up to 20% of the polymer mixture), causing an unpleasant sensation due to excessive deposition of hydrocolloid on the skin, when the cosmetic composition is used. Moreover the cosmetic composition is not transparent, and quite opaque. In this respect, as correctly explained in the detailed description of US 6180122, "gellan gum when used as the only gelling agent in a quantity exceeding 2% gives a composition which is solid but fragile and friable, and hence unusable for cosmetic and dermatological applications. Moreover, if the gellan gum quantity is increased, an opaque composition is obtained, hardly suitable for application to the skin". Only the presence of a quantity of hydrocolloid makes the composition usable, the rigidity of which can be modified by possibly adding to it one or more salts of the type stated in the introduction to the present description, but with the consequence that the presence of these salts makes the cosmetic compositions still more opaque and does not enable the visual characteristics of these cosmetic products to be highlighted. Moreover the excess of gellan gum and/or hydrocolloid makes the applicability of the cosmetic to the skin less satisfactory because of the presence of a greater total residue.

The main object of the present invention is to provide a solid cosmetic composition having high transparency or translucidity, which gives a sensation of freshness on application to the skin, has excellent make-up characteristics (i.e. able to be applied to the skin without leaving an excessive or unacceptable deposit of the composition on the skin), is easily extruded, and can be rehoused in appropriate containers ready for use.

These and further objects are attained by a solid cosmetic composition comprising
a) between 0.5% and 2% of gellan gum;
b) between 0.05% and 2% of a hydrocolloid chosen from the group consisting of xanthan gum, carboxymethyl cellulose, hydroxypropyl cellulose, hydroxypropylmethyl cellulose, hydroxymethyl cellulose, agar-agar, carrageen, alginates, carob gum, guar gum, gum arabic, karaya gum, tragacanth gum, ghatti gum, pectin, gelatin, caseinates, hydroxypropyl guar;
c) at least one salt chosen from between 0.01% and 1% of at least one salt of an alkaline-earth metal or between 0.05% and 5% of at least one alkaline metal salt, and between 0.01% and 5% of a mixture of alkaline metal and alkaline-earth metal salts, each within the aforestated limits, the constituent anions of said salts being chosen from the group consisting of carbonates and bicarbonates, sulphates, chlorides, phosphates, borates and acetates;
d) between 99.4% and 60% of water,
   all the indicated percentages being by weight on the total weight of the composition, which also comprises:
e) at least one cosmetically acceptable component chosen from the group consisting of preservatives, antioxidants, UV filters, emollients, pigments, colorants, surfactants, emulsifiers, oils, greases, perfumes, deodorants, antiperspirants, solvent extracts, powders, decorative particles, active principles, abrasives, whiteners, in a quantity sufficient to bring to 100 the total weight of the various components of the composition.

The present invention will be more apparent from the following nonlimiting examples of its implementation.

### EXAMPLE 1

### Preparation of a solid refreshing gelled composition

2 g of hydroxypropyl guar (known commercially as Jaguar HP 105) are dispersed under mechanical agitation in 940.5 g of deionized water. During dispersion of the powder, the mixture temperature is brought to 80°C. When a homogeneous phase is obtained, 16 g of gellan gum (of the type known commercially as Kelcogel F) are added slowly under agitation and mixing is continued until a homogeneous phase is obtained (5 min.). 2 g of xanthan gum (Rhodicare T) are then added to the mixture and mechanical mixing is continued until a homogeneous phase is obtained (5 min.). 3 g of Fenocombin (a preservative consisting of a mixture of phenoxyethanol and parabens), 5 g of phenoxyethanol and 2.5 g of sodium para methyl are added at 80°C, mixing with mechanical agitation for 3 min. 9 g of a 10% citric acid solution and 20 g of a 5% magnesium sulphate solution are added to the so obtained mixture. After 5 minutes of mixing, the composition obtained is poured at a temperature of 80°C into a stick-forming mechanism. When cold, the stick can be extruded by the stick-forming mechanism and can be coupled to a slider support.

The composition presents good make up characteristics, and does not leave visible residues on the skin. During application it leaves a pleasant fresh sensation.

### EXAMPLE 2

### Preparation of a solid transparent deodorant refreshing composition

2.5 g of hydroxypropyl guar (Jaguar HP 105) are dispersed under mechanical agitation in 859.5 g of deionized water. During dispersion of the powder, the mixture temperature is brought to 80°C. When a homogeneous phase is obtained, 18 g of gellan gum (Kelcogel F) are added slowly under agitation and mixing is continued until a homogeneous phase is obtained (5 min.). 2.5 g of xanthan gum (Rhodicare T) are then added and mechanical mixing is continued until a homogeneous phase is obtained (5 min.). 3 g of Fenocombin, 5 g of phenoxyethanol and 2.5 g of sodium para methyl are added at 80°C, mixing with mechanical agitation for 3 min.

In a second container, a second phase is prepared by slowly adding the following ingredients in the stated order: 15 g of perfume, 1 g of triclosan (known commercially as Irgasan DP 300), 20 g of dipropylene glycol, 25 g of PEG 40 - hydrogenated castor oil (Cremophor RH40), 5 g of sodium lauryl sarcosinate (Crodasinic LS30), 10 g of PEG 7-glyceryl cocoate (Glicerox HE) and 2 g of Cetereth-6 (Rolfor HT6). The mixture obtained is heated to a temperature of 80°C under slow stirring. When the solution is homogeneous the phase is added to the remaining product and mixed for 3 min. 9 g of a 10% citric acid solution and 20 g of a 5% magnesium sulphate solution are then added. After 5 minutes of mixing, the composition is poured at a temperature of 80°C into a stick-forming mechanism. When cold, the solid stick can be extruded by the stick forming mechanism and can be coupled to the sliding support.

The composition presents good make-up characteristics and does not leave visible traces on the skin. During application it leaves a pleasant fresh sensation.

### EXAMPLE 3

### Preparation of a solid transparent refreshing composition with decorative particles

2.2 g of hydroxypropyl guar (Jaguar HP 105) are dispersed under mechanical stirring in 940.3 g of deionized water. During dispersion of the powder, the mixture temperature is brought to 80°C. When a homogeneous phase is obtained, 19 g of gellan gum (Kelcogel F) are added slowly under agitation and mixing is continued until a homogeneous phase is obtained (5 min.). 3 g of xanthan gum (Rhodicare T) are then added and mixing is continued until a homogeneous phase is obtained (5 min.). 3 g of Fenocombin, 5 g of phenoxyethanol and 2.5 g of sodium para methyl are added at 80°C, mixing with mechanical stirring for 3 min. 2 g of decorative particles ("Reflects glittering gold"), 8 g of a 10% citric acid solution and 15 g of a 5% magnesium sulphate solution are added to the so obtained mixture. After 5 minutes of stirring, the composition obtained is poured at a temperature of 80°C into a stick-forming mechanism. When cold, such a solid stick can be extruded by the stick-forming mechanism and can be coupled to a slider support.

The composition presents good make-up characteristics, and when applied to the skin uniformly distributes the particles dispersed within it to create a luminous bright effect. During application it leaves a pleasant fresh sensation.

The decorative particles used in these applications can be of different dimensions and quantity. Their concentration can vary from 0.01% to 30% by weight depending on the effect to be obtained.

### EXAMPLE 4

### Preparation of a solid refreshing gel composition for dental use

2.1 g of hydroxypropyl guar (Jaguar HP 105) are dispersed under mechanical agitation in 897.4 g of deionized water. During dispersion of the powder, the mixture temperature is brought to 80°C. When a homogeneous phase is obtained, 19 g of gellan gum (Kelcogel F) are added slowly under stirring and mixing is continued until a homogeneous phase is obtained (5 min.). 3 g of xanthan gum (Rhodicare T) are then added and stirring is continued until a homogeneous phase is obtained (5 min.). 3 g of Fenocombin, 5 g of phenoxyethanol and 2.5 g of sodium para methyl are added at 80°C, mixing with mechanical agitation for 3 min. 40 g of abrasive silica (Tixosil 63), 5 g of sodium lauryl sarcosinate (Crodasinic LS95), 8 g of a 10% citric acid solution and 15 g of a 5% magnesium sulphate solution are added to the mixture obtained. After 5 minutes of stirring the composition is poured at a temperature of 80°C into a stick-forming mechanism. When cold, such a solid stick can be extruded by the stick forming mechanism and can be coupled to the sliding support.

The composition presents good make-up characteristics and performs a cleaning action when applied to the denture.

The silica used in these applications can be of different sizes and quantity. Its concentration can vary from 1% to 30% by weight according to the effect to be obtained.

## Claims

1. A solid cosmetic composition comprising
a) between 0.5% and 2% of gellan gum;
b) between 0.05% and 2% of a hydrocolloid chosen from the group consisting of xanthan gum, carboxymethyl cellulose, hydroxypropyl cellulose, hydroxypropylmethyl cellulose, hydroxymethyl cellulose, agar-agar, carrageen, alginates, carob gum, guar gum, gum arabic, karaya gum, tragacanth gum, ghatti gum, pectin, gelatin, caseinates, hydroxypropyl guar;
c) at least one salt chosen from between 0.01 % and 1% of at least one alkaline-earth metal salt between 0.05% and 5% of at least one alkaline metal salt, and between 0.01% and 5% of a mixture of alkaline metal and alkaline-earth metal salts, each within the aforestated limits, the constituent anions of said salts being chosen from the group consisting of carbonates and bicarbonates, sulphates, chlorides, phosphates, borates and acetates;
d) between 99.4% and 60% of water, all the indicated percentages being by weight on the total weight of the composition, which also comprises:
e) at least one cosmetically acceptable component chosen from the group consisting of preservatives, antioxidants, UV filters, emollients, pigments, colorants, surfactants, emulsifiers, oils, greases, perfumes, deodorants, antiperspirants, solvent extracts, powders, decorative particles, active principles, abrasives, whiteners, in a quantity sufficient to bring to 100 the total weight of the various components of the composition.

2. A composition as claimed in claim 1, contained in a rigid support with a device for its gradual extraction.

3. A composition as claimed in claim 1 or 2, usable as:
a. a cosmetic or dermatological product
b. a cosmetic product for personal hygiene: deodorant, antiperspirant, and the like.
c. a cosmetic product for make-up of the skin and/or cutaneous annexes
d. a product for the cure and/or treatment of the hair and/or other cutaneous annexes
e. a product for the cure and/or cleaning of the teeth.
